# EUROPEAN PATENT APPLICATION

(11) **EP 2 213 645 A1**
(43) Date of publication of application: **04.08.2010**
(21) Application number: 09001180.0
(22) Date of filing: 28.01.2009
(51) Int. Cl.: C07C 41/09, C07C 43/215, C07F 17/02

(54) **Process for the preparation of an allyl aryl ether by catalytic o-allylation**

(71) Applicant: Hexion Specialty Chemicals Research Belgium S.A., 1348 Ottignies Louvain-la-Neuve (BE)
(72) Inventor: Postma, Ron, 3196 KK Vondelingenplaat RT (NL)

(57) **Abstract**

The invention provides a process for the preparation of an allyl aryl ether comprising the O-allylation of an aromatic hydroxyl containing compound with an allyl source in the presence of a catalyst, wherein the catalyst is a transition metal complex with a phosphine ligand, and wherein the phosphine ligand is
- either a monodentate phosphine P(X)₃, wherein each X independently is a an aliphatic substituent having from 1 to 9 carbon atoms, a cycloaliphatic substituent having from 5 to 9 carbon atoms or aromatic substituent having from 6 to 9 carbon atoms, or wherein two substituents X together form a divalent alkylene group, with from 3 up to 6 carbon atoms,
- or a bidentate diphosphine X₂P-R-PX₂ wherein each X has the same meaning as defined for the monodentate phosphine , wherein the divalent alkylene group is attached to the same or different phosphorus atom(s), and R is a bridging group having at least 4 bridging atoms between the phosphorus atoms,

wherein the O-allylation is carried out in the presence of an acid in an amount of at least 0.1 mol% calculated on the aromatic hydroxyl containing compound.

This invention further provides a process for the preparation of epoxy resins wherein as intermediate use is made of the allyl aryl ethers prepared by the process of the invention.

## Description

### Technical Field

The present invention relates to a process for the preparation of an allyl aryl ether by catalytic O-allylation of aromatic hydroxyl containing compounds ("phenols"). This may be achieved by reaction of an allyl source such as allyl alcohol or a similar reactant in the presence of a suitable catalyst. The selective O-allylation over the C-allylation or isomerisation is achieved by the use of metal complexes with certain phosphine ligands in an acidic environment as catalyst.

### Background Art

Allyl ethers of polyphenols are useful for preparing epoxy compounds low in aliphatic halogen content. From US 4740330 it is known that the aromatic hydroxyl groups of aromatic hydroxyl-containing compounds can be O-allylated to greater than 95% conversion by reacting the alkali metal salt of said aromatic hydroxyl-containing compounds with an allyl halide such as allyl chloride in the presence of a polar aprotic solvent such as dimethylformamide. These compounds are useful in preparing epoxy resins containing a low halogen content. The process of this reference requires the use of an allyl halide and thus generates halide salts as by-products. From an environmental and atom-efficiency point of view, this route is not attractive.

Likewise US 7049388 discloses a process for manufacturing an alpha -dihydroxy derivative from an aryl allyl ether wherein such alpha -dihydroxy derivative can be used to prepare an alpha -halohydrin intermediate and an epoxy resin prepared therefrom including epoxidizing an alpha -halohydrin intermediate produced from a halide substitution of an alpha -dihydroxy derivative which has been obtained by a dihydroxylation reaction of an aryl allyl ether in the presence of an oxidant or in the presence of an oxidant and a catalyst. The starting material in the dihydroxylation process is an aryl allyl ether or mixture of aryl allyl ethers. In Example 1 the conversion of bisphenol A to bisphenol A diallyl ether is described, which was synthesized as described in US4740330 mentioned above. This process therefore suffers from the same disadvantages as that described in US4740330.

From **VAN DER DRIFT,** Robert C., et al. Two reactions of allylic alcohols catalysed by ruthenium cyclopentadienyl complexes with didentate phosphine ligands: isomerisation and ether formation. Journal of Moleular Catalysis A: Chemical. 2000, vo1.159, p.163-177. it is known that ethers may be formed from heterocoupling of allyl alcohol with aromatic alcohols. In particular [RuCICp(*o-*MeO-dppe)], wherein Cp stands for η⁵-cyclopentadienyl and *o*-MeO-dppe stands for 1,2-bis(di(*ortho*-methoxyphenyl)phosphino)ethane, has proven to be very active in ether formation. Thus phenol was converted into allyl phenyl ether; *p-*cresol was converted into allyl (*p*-methylphenyl) ether (AMPE); *p*-MeOphenol was converted into allyl (*p*-methoxyphenyl) ether; 2,4,6-trimethylphenol was converted into allyl (2,4,6-trimethylphenyl) ether and 2-naphthol was converted into allyl 2-naphtyl ether. Turnover frequencies (TOF) of up to 875 h⁻¹ were achieved. On the other hand, under the reaction conditions applied, at 100 degrees Centigrade, AMPE underwent a Claisen rearrangement to form a mixture of several ring allylated cresols and ethers. C-allylation, however, is undesirable.

In the same article and in a similar article by TROST, B., et al. J. Am. Chem. Soc.. 1993, vo1.115, p.2027-2036 certain ruthenium cyclopentadienyl complexes were reported to be efficient catalysts for the isomerisation of allyl alcohols into aldehydes. These catalysts, based on 1,4-bis(diphenylphosphino)butarie (dppb) and on two monodentate phosphine ligands (here triphenylphospine, or PPh₃), were found to be inactive for allylation, resulting in the preparation of propanal from allyl alcohol in an isomerisation reaction. Indeed, in the article of VAN DER DRIFT et al., these catalysts were believed to be completely inactive towards allylation, let alone O-allylation.

In SABURI, Hajime, et al. Catalytic Dehydrative Allylation of Alcohols. Angew. Chem., Int. Ed. Engl.. 2005, vol.44, p.1730-1732. the salt waste-free allylation of alcohols is described, using allyl alcohol as allyl source. Water is thus the only coproduct, making this dehydrative allylation both efficient, environmentally friendly and simple to operate. It too employs a cationic ruthenium cyclopentadienyl complex. On the other hand, the direct allylation of phenol with allyl alcohol (2-propen-1-ol), catalyzed by CpRuPF₆]-2-pyridinecarboxylic acid derivative combined systems was low, likely as a result of the low nucleophilicity of the phenol and the reversibility of the catalysis.

Not only Ruthenium has been used for etherification of allyl alcohols with phenols. In SATOH, Tetsuya, et al. Palladium-Catalyzed Etherification of Allyl Alcohols Using Phenols int he Presence of Titanium(IV) Isopropoxide. J. org. chem.. 1997, vol.62, p.4877-4879. the palladium-catalyzed nucleophilic substitution of allyl alcohols in the presence of titanium(IV) isopropoxide is described. The catalyst requires a stoichiometric amount of promoter to achieve O-allylation, generating large amounts of waste.

Likewise, in KIM, Hahn, et al. A Mild and Efficient Method for the Stereoselective Formation of C-O Bonds: Palladium-Catalyzed Allylic Etherification Using Zinc(II) Alkoxides. Organic Letters. 2002, vol.4, no.24, p.4369-4371. a highly chemo- and stereoselective palladium-catalyzed allylic etherification reaction is described. In this reference aliphatic alcohols are reacted with allylic acetates. Again, the catalyst requires stoichiometric amounts of zinc alkoxide as promoter, generating large amounts of waste. No information can be found on the O-allylation of aromatic alcohols.

In BRUNEAU, Christian, et al. Pentamethylcyclopentadienyl-Ruthenium Catalysts for Regio- and enantioselective allylation of Nucleophiles. Chem. Eur. J.. 2006, vol.12, p.5178-5187. the allylation of phenols with e.g., cinnamyl chloride or with allyl benzyl chloride was investigated. Various catalysts were tested. The yield of O-allylated product, however, is low; apparently the C-C allylation is highly favoured.

C-allylation is known from a series of references. For instance palladium-catalyzed C-allylation is known from KIMURA, Masanari, et al. Palladium-Catalyzed, Triethylborane-Promoted C-allylation of Naphthols and Benzene Polyols by Direct Use of Allyl Alcohols. Synthesis. 2006, vol.21, p.3611-3616. and KUNTZ, Emile, et al. Palladium TPPTS catalyst in water: C-allylation of phenol and guaiacol with allyl alcohol and novel isomerisation of allyl ethers of phenol and guaiacol. Journal of Molecular Catalysis A: Chemical. 2006, vol.244, p.124-138. Kimura describes the direct C-allylation of nathols and benzene polyols, using a combination of a catalytic amount of Pd(0) species and triethylborane as catalyst. Kuntz, on the other hand, produces the alkenylphenols through a fast transformation of ethers. Ignacia Fernández et al. discloses the C-allylation using dicationic Ru^{IV} Catalysts in FERNANDEZ, Ignacio, et al. High-Yield Ruthenium-Catalyzed Friedel-Crafts-Type Allylation Reactions Using Dicationic Ru(IV) Catalysts. Angew. Chem., Int. Ed. Engl.. 2006, vol.45, p.6386-6391. and FERNANDEZ, Ignacio, et al. Allylic Alcohols as Substrate for Ruthenium-Catalyzed C-C Coupling Allylation Reactions. Helvetica Chimica Acta. 2007, vol.90, p.271-276.

It should be noted that O-allylated products, if formed at all, are typically reversibly formed, while C-allylated products are produced irreversibly. C-allylation can thus be the predominant reaction because this yields the eventual thermodynamic end products, making it hard to produce allyl aryl ethers. The problem underlying the current invention is to improve the selective O-allylation in a manner that is environmentally friendly and highly atom-efficient, without generating saline waste. This problem has been solved, by use of certain ligands believed to be inactive towards C-allylation.

It should also be noted that bidentate phosphines and their synthesis have been disclosed in earlier patent references, such as EP 380162 on catalyst compositions suitable for the preparation of polymers of carbon monoxide and one or more olefinically unsaturated compounds. Likewise, in CA 2086285 a process is found for the preparation of vicinally-disubstituted bis(diorganophosphino)alkanes, -alkenes, or -arenes, which are prepared by reacting a secondary phosphine with a vicinally disubstituted dihaloalkane, -alkene or -arene. Moreover, in WO 0187899 bidentate ligands are described having a bivalent aliphatic bridging group containing from 2 to 6 atoms in the bridge, which is substituted with two or more substituents. The catalyst system comprises: a) a source of group VIII metal cations, b) a source of such a bidentate ligand, and c) a source of anions. Also described is the use of such a catalyst system in a process for the carbonylation of optionally substituted ethylenically or acetylenically unsaturated compounds by reaction with carbon monoxide and a co-reactant. None of these references, however, discuss the preparation of O-allylated products.

### Disclosure of Invention

Accordingly, the invention provides a process for the preparation of an allyl aryl ether comprising the O-allylation of an aromatic hydroxyl containing compound with an allyl source in the presence of a catalyst, wherein the catalyst is a complex of a transition metal M with a phosphine ligand and optionally comprising a coordinating species L and/or one or more counterions Y, and wherein the phosphine ligand is
- either a monodentate phosphine P(X)₃, wherein each X independently is a an aliphatic substituent having from 1 to 9 carbon atoms, or a cycloaliphatic substituent having from 5 to 9 carbon atoms or aromatic substituent having from 6 to 9 carbon atoms, or wherein two substituents X together form a divalent alkylene group, with from 3 up to 6 carbon atoms,
- or an bidentate diphosphine X₂P-R-PX₂ wherein each X has the same meaning as defined for the monodentate phosphine, wherein the divalent alkylene group is attached to the same or different phosphorus atom(s), and R is a bridging group having at least 4 bridging atoms between the phosphorus atoms, preferably a linear bridging group,
wherein the O-allylation is carried out in the presence of an acid in an amount of at least 0.1 mol% calculated on the aromatic hydroxyl containing compound.

More specifically, the invention provides a process for the preparation of an allyl phenyl ether using a ruthenium-cyclopentadienyl complex (Ru-Cp, wherein Cp is a cyclopentadienyl group or a substituted cyclopentadienyl group) as catalyst.

The allyl aryl ethers prepared by the process of the invention can be used, amongst others, as intermediate in the synthesis of epoxy resins. Hence the use of these ethers has also been claimed.

### Modes for Carrying Out the Invention

Where in this application reference is made to a catalyst, then such definition includes the precatalyst that is activated during the reaction, as well as the activating species in different oxidation states generated during the catalytic reaction. For instance, it is common to activate a Ru-Cp complex with silver tosylate (4-toluenesulfonate) to remove the strongly coordinating chloride anion present in the precatalyst. Instead of silver tosylate, also silver triflate (trifluoromethanesulfonate), silver tetrafluoroborate, silver hexafluorophosphate and similar non-coordinating silver salts may be used.

The aromatic hydroxyl containing compound is a phenolic compound having at least one aromatic nucleus and at least one hydroxyl group attached thereto. Unsubstituted phenol (C₆H₅OH) is the simplest species of this genus. 1,2- or 1,3-or 1,4-benzenediol and 1,3,5-benzene triol are the simplest species carrying more than one hydroxyl group. Moreover, the phenol may carry one or more inert substituents, such as e.g. *p*-cresol, 2,4,6-trimethylphenol or 4-*tert*-butylphenol. The aromatic nucleus may be part of a fused ring system, such as 1- or 2-naphthol, 1-, 2-, or 9-anthrol, which again may have more than 1 hydroxyl group (e.g., anthralin = 1,8,9-trihydroxyanthracene), or fused with a cycloaliphatic ring (s) such as for example indane, 1,2, 3,4-tetrahydronaphthalene and fluorene. Also suitable are phenolic compounds containing multinuclear aromatic rings, such as for example biphenyl, 2,2-diphenyl propane, bisphenylene oxide, tetrakis (1,1, 2,2-phenyl) ethane, stilbene, phenol-formaldehyde novolac, cresol-formaldehyde novolac, phenoldicyclopentadiene novolac and hyper-branched aromatic phenol dendrimers. The phenolic compound may also contain one or more heteroatoms such as for example O, N, S, Si, B or P; or any combination of these heteroatoms, such as for example, quinoxaline, thiophene and quinoline. Ar may also be a moiety containing mononuclear or multinuclear aromatic ring (s). Ar may also be a moiety containing mononuclear or multinuclear aromatic ring (s) fused with a cycloaliphatic ring (s) containing one or more heteroatoms such as for exampleO, N, S, Si, B or P; or any combination of these heteroatoms, such as for example, chroman, indoline and thioindane.

Of particular interest are phenolic compounds having 2 or more hydroxyl groups, which result in allyl aryl ethers that are multifunctional and hence suitable as intermediate in the synthesis of epoxy resins.

The process of the current invention aims at the production of allyl aryl ethers in an environmentally friendly and atom-efficient manner. As allyl source, allyl halides are preferably excluded, since these would result in the generation of a saline waste. To a lesser extent, also allyl acetates are preferably excluded. Rather, the preferred allyl source is either an allyl alcohol or an allyl ether. 2-Propen-1-ol is again the simplest species of an allyl alcohol. Other examples of suitable allyl alcohols include 3-buten-2-ol, cinnamyl alcohol (3-phenyl-2-propen-1-ol), and other alcohols of the general formula 1-R-2-propen-1-ol or 1-Ar-2-propen-1-ol or 1-RO-2-propen-1-ol or 1-ArO-2-propen-l-ol, or 3-R-2-propen-1-ol or 3-Ar-2-propen-1-ol or 3-RO-2-propen-1-ol or 3-ArO-2-propen-1-ol, wherein R represents an alkyl group, preferably of up to 6 carbon atoms, wherein O represents an oxygen atom, and wherein Ar represents an aryl group, preferably of up to 12 carbon atoms. These alcohols may be used by themselves, or in a mixture. Moreover, their diethers may be used, or ethers of a mixture of these alcohols. Moreover, a mixture of allyl alcohol(s) and allyl ether(s) may be used.

Preferably the allyl source is 2-propen-1-ol ("AA") or the ether thereof ("DAE"), or a combination of AA and DAE, most preferably AA.

The aromatic hydroxyl containing compound and the allyl source are preferably reacted in amounts sufficient to have at least one of the hydroxyl groups of the aromatic hydroxyl containing compound react with the allyl source. In case of a phenolic monool (e.g., phenol), and AA as the allyl source, the molar ratio between these reactants varies from 10:1 1 to 1:10, suitably from 5:1 to 1:5, preferably from 3:1 to 1:3, more preferably from 2:1 to 1:2. In case of DAE, the relative amount of aromatic hydroxyl containing compound is increased, as DAE may generate ideally two molecules of AA to react with the phenolic compound. In case of a phenolic polyol (e.g., bisphenol A or F) the relative amount of aromatic hydroxyl containing compound is decreased, corresponding to the higher hydroxyl content of said compound.

The catalyst systems of the current invention may also be used for the allylation of alkanols and/or aliphatic polyols.

The catalyst is a transition metal complex with either two monodentate phosphine ligands or a bidentate diphosphine ligand, and optionally comprising a coordinating species and/or a counterion, wherein M is a transition metal, L is a coordinating species, and Y is a counterion.

Suitable transition metals include ruthenium, rhodium, rhenium, palladium, iridium, tungsten, molybdenum, chromium, platinum, nickel, tin, copper, osmium, iron, and preferably from ruthenium, osmium, and iron and more preferably is ruthenium. Various coordinating species L may be used, subject to the selection of the transition metal and the transition state of the metal. It is reiterated that the transition state of the metal as active catalyst may and frequently is different from that of the precatalyst. Coordinating species may include carbonyl groups, indenyl, cyclopentadienyl and optionally substituted cyclopentadienyl groups. Indeed, in case of a ruthenium complex, the coordinating species is preferably cyclopentadienyl or a substituted cyclopentadienyl group (e.g., Cp* = pentamethylcyclopentadienyl).

From VAN DER DRIFT et al., mentioned supra, bidentate ligands and Ru-Cp complexes based thereon are known. The diphosphines used as ligands all have unsubstituted aliphatic bridging groups between the phosphorus atoms. Surprisingly, it has been discovered that in the presence of strong acid, for instance p-toluenesulfonic acid, isomerization can be efficiently blocked and the complexes become very active in catalyzing O-allylation reactions.

In case of a bidentate diphosphine, the bridge may comprise four or more bridging atoms. Preferably, the bridge comprises 4 carbon atoms, i.e., a 1,4-butylene group. The bridging group may be substituted or branched, but preferably is linear. The substituents X attached to the phosphorus atom(s) each independently may be selected from alkyl groups, with preferably up to 6 carbon atoms, or cycloakyl groups having from 5 to 9 carbon atoms or aryl groups having from 6 to 9 carbon atoms. The cycloalkyl and/or aryl group may be substituted with one or more alkyl groups having up to 6 carbon atoms. Two substituents X may also together form a divalent alkylene group, with from 3 up to 6 carbon atoms, attached to the same or different phosphorus atom(s). The two phosphorus atoms may therefore be part of a 1,5-diphosphacyclooctane or similar substituted heterocycle. These substituents X may also comprise one or more functional groups and/or have one or more heteroatoms as part of the group. Particularly suitable substituents X include phenyl, ortho-methoxyphenyl, ortho-ethoxylphenyl, and cyclohexyl.

Preferably the substituents X are identical.

Illustrative examples of suitable ligands therefore include.
- 1,4-bis(diphenylphosphino)-butane (dppb)
- 1,5-bis(diphenylphosphino)-pentane (dpppt) and
- triphenylphosphine.

The catalyst is present during the reaction in a catalytically effective amount, preferably in an amount of from about 0.005 to about 200 mmoles of metal per mole of hydroxyl group(s) in the phenolic compound. More preferably, the catalyst is used in an amount of from about 0.05 to 20 mmoles per mole of hydroxyl group(s) in the phenolic compound. Indeed, for reasonable activity, most preferably small amounts of catalyst are used, such as about 0.1 mmole per mole of hydroxyl group(s) in the phenolic compound.

As mentioned before, the catalyst may need to be activated to perform the O-allylation. Ru-Cp complexes typically have a strongly coordinating chloride anion when obtained as precatalyst, which may be removed with silver tosylate, silver triflate, or other non-coordinating anions such as silver tetrafluoroborate, silver hexafluorophosphate to create a vacant site for substrate coordination.

The catalyst may be immobilized, resulting in a heterogeneous catalyst. For instance, the catalyst (metal complex) can be immobilized on an ion-exchange resin with sulfonic acid groups. Examples of suitable resins include for example DOWEX™ WX4, Amberlyst™ 15 or Nation™ NR 50.

The O-allylation products may be prepared in a continuous reaction or in a batch reaction.

In the process of the current invention, the O-allylation reaction is performed in the presence of an acid. Thus, whereas VAN DER DRIFT et al, mentioned supra, found the selectivity towards C-allylation was improved, which would therefore be detrimental to the balance of activity and selectivity, the inventors surprisingly found a high activity and selectivity in the process of the invention when performed in the presence of an acid. Thus, the presence of 0.1 mol% to 25 mol%, preferably 0.15 to 20 mol%, more preferably 0.2 to 10 mol% and most preferably 1 to 5 mol% of acid calculated on the amount of phenolic compound increases the O-allylation reaction rate whilst avoiding the preparation of C-allylated products. The acid may be selected on the basis of its logarithmic acid dissociation constant, pKa. The acid is preferably selected from the organic acids with a pKa < 5, preferably < 3. Suitable acids include acetic acid and triflic acid and such, an in particular those acids whose silver salt have been used to activate the catalyst. The most preferred acid is p-toluenesulfonic acid.

The reaction is preferably carried out in an organic solvent. Although solvents like diethylene glycol dimethyl ether (diglyme) may be used, the solvent is more preferably an apolar solvent. The selection typically is done on the basis of the availability, cost and acceptability for environmental and health issues. The solvent has an effect on the total conversion rate of the reaction and the selectivity of the allylation (O-allylation versus C-allylation). The organic solvents may be selected from aromatic or aliphatic hydrocarbons, with the preferred solvent being toluene and the most preferred solvent being heptane.

The reactions are preferably conducted at a temperature between 40 and 150°C.

For catalysts based on a bidentate diphosphine ligand the reactions are preferably conducted between 80 and 130°C, whilst for catalysts based on a monodentate phosphine ligand the temperature is preferably between 50 and 80°C.

The following examples will more fully illustrate selected embodiments of this invention. All parts, percentages and proportions referred to herein and in the appended claims are by mol unless otherwise indicated.

### Examples

**Table 1. ligand abbreviations**

| Inventive | Comparative | Compound |
|---|---|---|
| | dppp | 1,3-bis(diphenylphosphino)propane |
| dppb | | 1,4-bis(diphenylphosphino)butane |
| PPh₃ | | triphenylphosphine |
| | | |

### Ligand synthesis

The ligands were commercially available from Acros.

The ruthenium complexes [RuCpCl(PPh₃)₂]¹; and [RuCpCI(dppb)](ref VAN DER

DRIFT et al)were synthesized as reported.

### Reaction of 4-tert-butylphenol with allyl alcohol under catalysis of different complexes

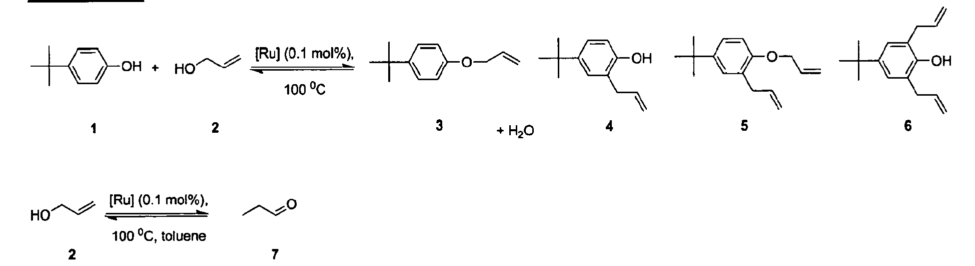

Allylation reactions were carried out in the manner shown above, at a molar ratio of 4-*tert*-butylphenol/allyl alcohol/catalyst/AgOTs = 1000/1000/1/2, using toluene as solvent, at 100 °C for 3 hours. The products were analyzed by GC, identified by GC-MS and NMR spectroscopy and the results are reported below.

¹ Bruce, M. I.; Wong, F. S.; Skelton, B. W.; White, A. H. J. Chem. Soc.-Dalton Trans. 1981, 1398-1405.

### Comparative Example 1

**[RuCpCl(PPh₃)₂].** 2.5 mmol of 4-*tert*-butylphenol, 2.5 µmol of [RuCpCl(PPh₃)₂)] and 5 µmol of AgOTs were charged into the reaction vessel and flushed with argon. Degassed and dried toluene was added (3 ml) and the mixture was stirred for five minutes. Allyl alcohol was added (2.5 mmol) and the reaction was stirred for 3 hours at 100 °C. Conversion of **1**: 0%. Meanwhile the conversion of **2** into **7** is 100%.

### Comparative Example 2

**[RuCpCl(dppb)].** As example 1, but with [RuCpCl(dppb)] instead of [RuCpCl(PPh₃)₂] Conversion of **1:** 0%. (See above) Again, the conversion of **2** into 7 is 100%.

### Example 3

**[RuCpCl(PPh₃)₂].** As example 1, but with addition of 5.0 µmol of *p*-toluenesulfonic acid. Conversion of **1:** 13%. Selectivity for: **3** 42%; **4** 58%.

### Example 4

**[RuCpCl(dppb)].** As example 2, but with addition of 5.0 µmol of *p*-toluenesulfonic acid. Conversion of **1:** 58%. Selectivity for: **3** 83%; **4** 13%; **5** 4%.

### Example 5

**[RuCpCl(dppb)].** As example 4, but with 5.0 instead of 2.5 mmol of allyl alcohol. Conversion of **1:** 56%. Selectivity for: **3** 85%; **4** 13%; **5** 2%.

### Example 6

**[RuCpCl(dppb)].** As example 5, but at 80 instead of 100°C. Conversion of **1:** 30%. Selectivity for: **3** 91 %; **4** 9%.

### Example 7

**[RuCpCl(dppb)].** As example 5, but at 60 °C. Conversion of **1:** 8%. Selectivity for: **3** 100%.

### Example 8

**[RuCpCl(PPh₃)₂].** As example 7, but with [RuCpCl(PPh₃)₂] in stead of [RuCpCl(dppb)]. Conversion of **1:** 9%. Selectivity for: **3** 100%.

### Example 9

**[RuCpCl(PPh₃)₂].** As example 8, but with 50 µmol of *p*-toluenesulfonic acid and after 24 hours. Conversion of **1**: 84%. Selectivity for: **3** 91%; **4** 4%; **5** 5%.

### Example 10

**[RuCpCl(PPh₃)₂].** As example 8, but at with 125 µmol of *p*-toluenesulfonic acid. Conversion of **1:** 66%. Selectivity for: **3** 75%; **4** 18%; **5** 4%; **6** 2%.

### Comparative example 11.

As example 4, but with **[RuCpCl(dppp)]** instead of [RuCpCl(dppb)]. Conversion of **1:** 80%. Selectivity for: **3** 2%; **4** 69%; **5** 4%; **6** 25%.

### Conclusions/observations

Comparative examples 1 and 2 are without acid. The conversion of 1 is 0%. Examples 3 and 4 are similar to Comparative examples 1 and 2, but carried out in the presence of an acid (2 eq.) The conversion is improved, and the selectivity (in particular using dppb) is good.

Examples 5, 6 and 7 are similar to Example 4, but at different temperature conditions. From these Examples it may be concluded that [RuCpCl(dppb)] is a good catalyst at temperatures of 60 to 100 °C.

Examples 8-10 are done with [RuCpCl(PPh₃)₂] as catalyst. These examples indicate that [RuCpCl(PPh₃)₂] is a good catalyst at temperatures in the range of 60 to 80°C, and preferably at a slightly elevated amount of acid. Indeed, the examples illustrate that the catalyst without acid lead to the 100% conversion of the allyl alcohol into propionaldehyde, which reaction is efficiently blocked by using the catalyst in the presence of an acid.

Finally, Example 11 illustrates that the positive effect of the acid on the catalyst is related to the length of the bridge between the phosphorus atoms when using a bidentate diphosphine ligand in the catalyst. Thus, when a bidentate diphosphine having only 3 atoms in the bridge is used, selective O-allylation is not achieved.

### Industrial Application

Intermediate for pharmaceuticals, e.g., wherein the O-allylation reaction is applied to provide a protecting group on a hydroxyl functionality in multistep synthesis of certain fine and/or pharmaceutical compounds.

Intermediate for epoxy resins

## Claims

1. A process for the preparation of an allyl aryl ether comprising the O-allylation of an aromatic hydroxyl containing compound with an allyl source in the presence of a catalyst, wherein the catalyst is a complex of transition metal M with a phosphine ligand, and wherein the phosphine ligand is
- either a monodentate phosphine P(X)₃, wherein each X independently is a an aliphatic substituent having from 1 to 9 carbon atoms, or a cycloaliphatic substituent having from 5 to 9 carbon atoms or aromatic substituent having from 6 to 9 carbon atoms, or wherein two substituents X together form a divalent alkylene group, with from 3 up to 6 carbon atoms,
- or an bidentate diphosphine X₂P-R-PX₂ wherein each X has the same meaning as defined for the monodentate phosphine, wherein the divalent alkylene group is attached to the same or different phosphorus atom(s), and R is a bridging group having at least 4 bridging atoms between the phosphorus atoms,
wherein the O-allylation is carried out in the presence of an acid in an amount of at least 0.1 mol% calculated on the aromatic hydroxyl containing compound.

2. The process of claim 1 wherein M is selected from the transition metals ruthenium, rhodium, rhenium, palladium, iridium, tungsten, molybdenum, chromium, platinum, nickel, tin, copper, osmium, iron, and preferably from ruthenium, osmium, and iron and more preferably is ruthenium.

3. The process of claim 1 or 2, wherein a ruthenium-cyclopentadienyl complex (Ru-Cp, wherein Cp is a cyclopentadienyl group or a substituted cyclopentadienyl group) is used as catalyst.

4. The process of any one of claims 1 to 3, wherein a bidentate diphosphine ligand is used having four atoms in the bridge, preferably 4 carbon atoms.

5. The process of any one of claims 1 to 4, wherein each X independently is selected from: alkyl groups, with preferably up to 6 carbon atoms; aryl groups with preferably up to 9 carbon atoms; or divalent alkenyl groups, with from 3 up to 6 carbon atoms, attached to the same or different phosphorus atom, preferably phenyl, ortho-methoxyphenyl, ortho-ethoxylphenyl, and cyclohexyl.

6. The process of any one of claims 1 to 5, wherein the ligand is one of: 1,4-bis(phenylphosphino)-butane (dppb); or triphenylphosphine.

7. The process of any one of claims 1 to 6, wherein the catalyst is present during the reaction in a catalytically effective amount, preferably in an amount of from about 0.005 to about 200 mmoles of metal per mole of hydroxyl group(s) in the aromatic hydroxyl containing compound.

8. The process of any one of claims 1 to 7, wherein the aromatic hydroxyl containing compound is a phenolic compound having at least one aromatic nucleus and at least one hydroxyl group attached thereto, or a mixture of such compounds, which compound or mixture of compounds comprises substituted or unsubstituted phenols, aromatic hydroxyl containing compounds having a fused ring system, aromatic hydroxyl containing compounds containing multiple aromatic rings, and aromatic hydroxyl containing compounds contain one or more heteroatoms selected from O, N, S, Si, B or P; or any combination of these heteroatoms.

9. The process of any one of claims 1 to 8, wherein the allyl source is an allyl alcohol or mixture of allyl alcohols of the general formulae
1-R-2-propen-1-ol or
1-Ar-2-propen-1-ol or
1-RO-2-propen-1-ol or
1-ArO-2-propen-1-ol, or
3-R-2-propen-1-ol or
3-Ar-2-propen-1-ol or
3-RO-2-propen-1-ol or
3-ArO-2-propen-1-ol,
wherein R represents an alkyl group, preferably of up to 6 carbon atoms,
wherein O represents an oxygen atom, and wherein Ar represents an aryl group, preferably of up to 12 carbon atoms, and/or an ether of the allyl alcohol or allyl alcohols, preferably 2-propen-1-ol ("AA") or the ether thereof ("DAE") or the mixture of AA and DAE, preferably AA.

10. The process of any one of claims 1 to 9, wherein the allyl alcohol and the aromatic hydroxyl containing compound are used in a molar ratio of allyl alcohol versus the aromatic hydroxyl-containing compound of from 1:10 to 10:1, preferably of from 1:5 to 5:1, more preferably of from 1:2 to 2:1, most preferably about 1:1.

11. The process of any one of claims 1 to 10, wherein an acid with pKa < 5, preferably a pKa < 3, preferably selected from acetic acid, triflic acid and p-toluenesulfonic acid, more preferably p-toluenesulfonic acid is present in an amount of from 0.1 mol% to 25 mol%, preferably 0.15 to 20 mol%, more preferably 0.2 to 10 mol% and most preferably 1 to 5 mol% calculated on the amount aromatic hydroxyl containing compound used.

12. The process of any one of claims 1 to 11, conducted at a temperature between 40 and 150°C, preferably between 60 and 100°C when a bidentate diphosphine ligand is used or preferably between 60 and 80°C when a monodentate phosphine ligand is used.

13. The process of any one of claims 1 to 12, wherein the catalyst is immobilized, preferably on an ion-exchange resin.

14. Process for the preparation of epoxy resins wherein as intermediate use is made of the allyl aryl ethers prepared by the process of any one of claims 1 to 13.
